# NEUE EUROPÄISCHE PATENTSCHRIFT

(11) **EP 1 689 370 B2**
(45) Veröffentlichungstag und Bekanntmachung des Hinweises auf die Entscheidung über den Einspruch: **14.09.2016**
(45) Hinweis auf die Patenterteilung: 13.02.2008
(21) Anmeldenummer: 04797879.6
(22) Anmeldetag: 13.11.2004
(51) Int. Cl.: A61K 9/20, A61P 7/02, A61K 31/5377

(54) **VERFAHREN ZUR HERSTELLUNG EINER FESTEN, ORAL APPLIZIERBAREN PHARMAZEUTISCHEN ZUSAMMENSETZUNG Mit 5-Chlor-N ( { (5-2-oxo-3- [4- (3-oxo-4-morpholinyl)-phenyl]-1, 3- oxazolidin-5-yl}-methyl)-2-thiophencarboxamid**
METHOD FOR THE PRODUCTION OF A SOLID, ORALLY APPLICABLE PHARMACEUTICAL COMPOSITION COMPRISING 5-Chlor-N ( { (5-2-oxo-3- [4- (3-oxo-4-morpholinyl)-phenyl]-1, 3- oxazolidin-5-yl}-methyl)-2-thiophencarboxamide
PROCEDE POUR PRODUIRE UNE COMPOSITION PHARMACEUTIQUE SOLIDE A APPLICATION ORALE COMPRENANT DU 5-Chlor-N ( { (5-2-oxo-3- [4- (3-oxo-4-morpholinyl)-phenyl]-1, 3- oxazolidin-5-yl}-methyl)-2-thiophencarboxamide

(30) Priorität: 27.11.2003 DE 10355461
(43) Veröffentlichungstag der Anmeldung: 16.08.2006
(73) Patentinhaber: Bayer Intellectual Property GmbH, 40789 Monheim (DE)
(72) Erfinder: BENKE, Klaus, 51465 Bergisch Gladbach (DE)
(74) Vertreter: von Renesse, Dorothea
(86) Internationale Anmeldenummer: PCT/EP2004/012897
(87) Internationale Veröffentlichungsnummer: WO 2005/060940

(56) Entgegenhaltungen:
- WO-A-01/47919
- WO-A-03/035133

## Beschreibung

Die vorliegende Erfindung betrifft ein Verfahren zur Herstellung einer festen, oral applizierbaren pharmazeutischen Zusammensetzung, enthaltend 5-Chlor-*N*-({(5*S*)-2-oxa-3-[4-(3-oxo-4-morpholinyl)-phenyl]-1,3-oxazolidin-5-yl}-methyl)-2-thiophencarboxamid in hydrophilisierter Form sowie ihre Verwendung zur Prophylaxe und/oder Behandlung von Erkrankungen.

5-Chlor-*N*-({(5*S*)-2-oxo-3-[4-(3-oxo-4-morpholinyl)-phenyl]-1,3-oxazolidin-5-yl}-methyl)-2-thiopliencar-boxamid (I) ist ein niedermolekularer, oral applizierbarer Inhibitor des Blutgerinnungsfaktors Xa, der zur Prophylaxe und/ oder Behandlung verschiedener thromboembolischer Erlaankungen eingesetzt werden kann (siehe hierzu WO-A 01/47919, deren Offenbarung hiermit durch Bezugnahme eingeschlossen ist). Wenn im folgenden vom Wirkstoff (I) die Rede ist, so sind dabei alle Modifikationen von 5-Chlor-*N*-({(5*S*)-2-oxo-3-[4-(3-oxo-4-morpholinyl)-phenyl]-1,3-oxazoli-din-5-yl}-methyl)-2-thiophencarboxamid (I) sowie die jeweiligen Hydrate mit umfasst.

Der Wirkstoff (I) weist eine relativ schlechte Wasserlöslichkeit auf (ca. 7 mg/L). Dadurch können sich Schwierigkeiten bei der oralen Bioverfügbarkeit sowie eine erhöhte biologische Variabilität der Absorptionsrate ergeben.

Zur Erhöhung der oralen Bioverfügbarkeit sind in der Vergangenheit verschiedene Konzepte beschrieben worden:

So werden häufig Lösungen von Wirkstoffen angewendet, die beispielsweise in Weichgelatinekapseln abgefüllt werden können. Aufgrund der schlechten Löslichkeit des Wirkstoffes (I) in den für diesen Zweck geeigneten Lösungsmitteln ist diese Option im vorliegenden Fall aber nicht anwendbar, da in der notwendigen Dosisstärke Kapselgrößen resultieren würden, die nicht mehr schluckbar sind.

Ein alternatives Verfahren stellt die Amorphisierung des Wirkstoffes dar. Hierbei erweist sich sowohl die Lösungsmethode als problematisch, da der Wirkstoff (I) auch in pharmazeutisch akzeptablen Lösemittel wie Ethanol oder Aceton schlecht löslich ist. Auch eine Amorphisierung des Wirkstoffes über die Schmelzmethode ist wegen des hohen Wirkstoff-Schmelzpunktes (ca. 230°C) ungünstig, da ein unerwünscht hoher Anteil von Abbaukomponenten während der Herstellung entsteht.

Weiterhin ist ein Verfahren zur Hydrophilisierung von hydrophoben Wirkstoffen am Beispiel von Hexobarbital und Phenytoin beschrieben worden (Lerk, Lagas, Fell, Nauta, Journal of Pharmaceutical Sciences Vol. 67, No. 7, July 1978, 935 - 939: "Effect of Hydrophilization of Hydrophobic Drugs on Release Rate from Capsules"; Lerk, Lagas, Lie-A-Huen, Broersma, Zuurman, Journal of Pharmaceutical Sciences Vol. 68, No. 5, May 1979, 6,34-638: "In Vitro and In Vivo Availability of Hydrophilized Phenytoin from Capsules"). Die WirkstofReilchen werden hierbei in einem Mischer unter weitgehender Vermeidung eines Agglomerationsschrittes mit einer Methyl- bzw. Hydroxyethylcellulose-Lösung vermischt und dann getrocknet. Der so erhaltene Wirkstoff wird anschließend ohne weitere Behandlung in Hartgelatinekapseln abgefüllt.

Überraschenderweise wurde nun gefunden, dass eine spezielle Behandlung der Oberfläche des Wirkstoffes (I) im Rahmen der Feuchtgranulation ein verbessertes Absorptionsverhalten bewirkt wobei als Feuchtgranulationsmethode die Wirbelschichtgranulation verwendet wird. Die Verwendung des Wirkstoffes (I) in hydrophilisierter Form bei der Herstellung von festen, oral applizierbaren pharmazeutischen Zusammensetzungen führt zu einer signifikanten Erhöhung der Bioverfügbarkeit der so erhaltenen Formulierung.

Gegenstand der vorliegenden Erfindung ist ein Verfahren zur Herstellung einer festen, oral applizierbaren pharmazeutischen Zusammensetzung enthaltend 5-Chlor-*N*-({(5*S*)-2-oxo-3-[4-(3-oxo-4-morpholinyl)-phenyl]-1,3-oxa-zolidin-5-yl}-methyl)-2-thiophencarboxamid in hydrophilisierter Form, wobei
(a) zunächst ein den Wirkstoff (I) in hydrophilisierter Form enthaltendes Granulat durch Feuchtgranulation hergestellt wird
(b) und das Granulat dann, gegebenenfalls unter Zusatz pharmazeutisch geeigneter Zusatzstoffe, in die pharmazeutische Zusammensetzung überführt wird, wobei als Feuchtgranulationsmethode die Wirbelschichtgranulation verwendet wird.

Bei der Feuchtgranulation kann der Wirkstoff (I) entweder als Feststoff in der Vormischung (Vorlage) vorgelegt werden oder er wird in der Granulierflüssigkeit suspendiert. Bevorzugt wird der Wirkstoff (I) in der Granulierflüssigkeit suspendiert in die Feuchtgranulation eingetragen (Suspensionsverfahren).

In einer bevorzugten Ausführungsform der vorliegenden Erfindung wird der Wirkstoff (I) in kristalliner Form eingesetzt.

In einer besonders bevorzugten Ausführungsform der vorliegenden Erfindung wird der kristalline Wirkstoff (I) in mikronisierter Form eingesetzt. Der Wirkstoff (I) besitzt dabei vorzugsweise eine mittlere Partikelgröße X₅₀ kleiner 10 µm, insbesondere zwischen 1 und 8 µm sowie X₉₀ (90 %-Anteil) kleiner 20 µm, insbesondere kleiner 15 µm.

Die erfindungsgemäß verwendete Granulierflüssigkeit enthält ein Lösungsmittel, ein hydrophiles Bindemittel und gegebenenfalls ein Netzmittel. Das hydrophile Bindemittel ist dabei in der Granulierflüssigkeit dispergiert oder vorzugsweise darin gelöst.

Als Lösungsmittel der Granulierflüssigkeit können organische Lösungsmittel, wie beispielsweise Ethanol oder Aceton, oder Wasser oder Gemische davon verwendet werden. Bevorzugt wird Wasser als Lösungsmittel verwendet.

Als hydrophile Bindemittel der Granulierflüssigkeit werden pharmazeutisch geeignete hydrophile Zusatzstoffe eingesetzt, vorzugsweise solche, die sich im Lösungsmittel der Granulierflüssigkeit lösen.

Vorzugsweise werden dabei hydrophile Polymere wie beispielsweise Hydroxypropylmethylcellulose (HPMC), Carboxymethylcellulose (Natrium- und Calciumsalze), Ethylcellulose, Methylcellulose, Hydroxyethylcellulose, Ethylhydroxyethylcellulose, Hydroxypropylcellulose (HPC), L-HPC (niedrigsubstituierte HPC), Polyvinylpyrrolidon, Polyvinylalkohol, Polymere der Acrylsäure und deren Salze, Vinylpyrrolidon-Vinylacetat-Copolymere (beispielsweise Kollidon^{®} VA64, BASF), Gelatine, Guargummi, partiell hydrolisierte Stärke, Alginate oder Xanthan eingesetzt. Besonders bevorzugt wird HPMC als hydrophiles Bindemittel eingesetzt.

Das hydrophile Bindemittel kann dabei in einer Konzentration von 1 bis 15 % (bezogen auf die Gesamtmasse der pharmazeutischen Zusammensetzung), vorzugsweise von 1 bis 8 % enthalten sein.

Als gegebenenfalls vorhandene Netzmittel der Granulierflüssigkeit werden pharmazeutisch geeignete Netzmittel (Tenside) eingesetzt. Beispielsweise seien genannt:

Natriumsalze von Fettalkoholsulfaten wie Natriumlaurylsulfat, Sulfosuccinate wie Natriumdioctylsulfosuccinat, partielle Fettsäureester mehrwertiger Alkohole wie Glycerinmonostearat, partielle Fettsäureester des Sorbitans wie Sorbitanmonolaurat, partielle Fettsäureester des Polyhydroxyethylensorbitans wie Polyethylenglycol-Sorbitan-monolaurat, -monostearat oder-monooleat, Polyhydroxyethylen-Fett-alkoholether, Polyhydroxyethylen-Fettsäureester, Ethylenoxid-Propylenoxid-Blockcopolymere (Pluronic^{®}) oder ethoxylierte Triglyceride. Bevorzugt wird Natriumlaurylsulfat als Netzmittel eingesetzt.

Das Netzmittel wird bei Bedarf in einer Konzentration von 0.1 bis 5 % (bezogen auf die Gesamtmasse der pharmazeutischen Zusammensetzung), vorzugsweise von 0.1 bis 2 % eingesetzt.

In der Vormischung (Vorlage) der Feuchtgranulation sind weitere pharmazeutisch geeignete Zusatzstoffe enthalten. Beispielsweise seien genannt:
- Füllstoffe und Trockenbindemittel wie Cellulosepulver, mikrokristalline Cellulose, verkieselte mikrokristalline Cellulose, Dicalciumphosphat, Tricalciumphosphat, Magnesiumtrisilikat, Mannitol, Maltitol, Sorbitol, Xylitol, Laktose (wasserfrei oder als Hydrat, beispielsweise Monohydrat), Dextrose, Maltose, Saccharose, Glucose, Fructose oder Maltodextrine
- Zerfallsförderer (Sprengmittel) wie Carboxymethylcellulose, Croscarmellose (quervernetzte Carboxymethylcellulose), Crospovidone (quervernetztes Polyvinylpyrrolidon), L-HPC (niedrigsubstituierte Hydroxypropylcellulose), Natriumcarboxymethylstärke, Natriumglykolat der Kartoffelstärke, partiell hydrolisierte Stärke, Weizenstärke, Maisstärke, Reisstärke oder Kartoffelstärke

Im Fall von Tablettenformulierungen mit modifizierter (verzögerter) Wirkstofffreisetzung können statt der Zerfallsförderer (Sprengmittel) Stoffe enthalten sein, die die Freisetzungsrate beeinflussen. Beispielsweise seien genannt: Hydroxypropylcellulose, Hydroxypropylmethylcellulose, Methylcellulose, Ethylcellulose, Carboxymethylcellulose, Galaktomannan, Xanthan, Glyceride, Wachse, Acryl- und/oder Methacrylsäureester-Copolymerisate mit Trimethylammoniummethylacrylat, Copolymerisate von Dimethylaminomethacrylsäure und neutralen Methacrylsäureestern, Polymerisate von Methacrylsäure oder Methacrylsäureestern, Acrylsäureethylester-Methacrylsäuremethylester-Copolymerisate oder Methacrylsäure-Acrylsäuremethylester-Copolymerisate.

Das im Verfahrensschritt (a) erhaltene Granulat wird anschließend im Verfahrensschritt (b) in die erfindungsgemäße pharmazeutische Zusammensetzung überführt.

Der Verfahrensschritt (b) umfasst beispielsweise Tablettieren, Abfüllen in Kapseln, vorzugsweise Hartgelatinekapseln, oder Abfüllen als Sachets, jeweils nach üblichen, dem Fachmann geläufigen Methoden, gegebenenfalls unter Zusatz weiterer pharmazeutisch geeigneter Zusatzstoffe.

Als pharmazeutisch geeignete Zusatzstoffe seien beispielsweise genannt:
- Schmier-, Gleit-, Fließregulienmgsmittel wie Fumarsäure, Staearinsäure, Magnesiumstearat, Calciumstearat, Natriumstearylfumarat, höhermolekulare Fettalkohole, Polyethylenglykole, Stärke (Weizen-, Reis,- Mais- oder Kartoffelstärke), Talkum, hochdisperses (kolloidales) Siliciumdioxid, Magnesiumoxid, Magnesiumcarbonat oder Calciumsilikat
- Zerfallsförderer (Sprengmittel) wie Carboxymethylcellulose, Croscarmellose (quervernetzte Carboxymethylcellulose), Crospovidone (quervernetztes Polyvinylpyrrolidon), L-HPC (niedrigsubstituierte Hydroxypropylcellulose), Natriumcarboxymethylstärke, partiell hydrolisierte Stärke, Weizenstärke, Maisstärke, Reisstärke oder Kartoffelstärke

Weiterer Gegenstand der vorliegenden Erfindung ist eine feste, oral applizierbare pharmazeutische Zusammensetzung, nach Anspruch 6 oder 7 enthaltend 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) in hydrophilisierter Form.

Die erfindungsgemäße feste, oral applizierbare pharmazeutische Zusammensetzung umfasst beispielhaft und vorzugsweise Granulate, mit Granulat gefüllte Hartgelatinekapseln oder Sachets sowie den Wirkstoff (I) schnell oder modifiziert (verzögert) freisetzende Tabletten. Bevorzugt sind Tabletten, insbesondere den Wirkstoff (I) schnell freisetzende Tabletten. Im Rahmen der vorliegenden Erfindung sind schnellfreisetzende Tabletten insbesondere solche, die gemäß USP-Freisetzungsmethode mit Apparatur 2 (Paddle), wie im experimentellen Teil in Kapitel 4.2.2. beschrieben, einen Q-Wert (30 Minuten) von 75 % besitzen.

Der Wirkstoff (I) kann in der erfindungsgemäßen pharmazeutischen Zusammensetzung in einer Konzentration von 0,1 bis 60 %, vorzugsweise in einer Konzentration von 1 bis 40 %, bezogen auf die Gesamtmasse der Formulierung, vorliegen. Hierbei beträgt die Dosis des Wirkstoffes (I) vorzugsweise 1 bis 100 mg.

Gegebenenfalls werden die erfindungsgemäßen Granulate oder Tabletten in einem weiteren Schritt unter üblichen, dem Fachmann geläufigen Bedingungen lackiert. Die Lackierung erfolgt unter Zusatz von üblichen, dem Fachmann geläufigen Lackier- und Filmbildemitteln wie Hydroxypropylcellulose, Hydroxypropylmethylcellulose, Ethyl-cellulose, Polyvinylpyrrolidon, Vinylpyrrolidon-Vinylacetat-Copolymere (beispielsweise Kollidon^{®} VA64, BASF), Schellack, Acryl- und/oder Methacrylsäureester-Copolymerisate mit Trimethylammoniummethylacrylat, Copolymerisate von Dimethylaminomethacrylsäure und neutralen Methacrylsäureestern, Polymerisate von Methacrylsäure oder Methacrylsäureestern, Acrylsäureethylester-Methacrylsäuremethylester-Copolymerisate, Methacrylsäure-Acrylsäuremethylester-Copolymerisate, Propylenglykol, Polyethylenglykol, Glycerintriacetat, Triethylcitrat und/oder Farbzusätzen/Pigmenten wie beispielsweise Titandioxid, Eisenoxide, Indigotin oder geeigneter Farblacke.

Weiterer Gegenstand der vorliegenden Erfindung ist die Verwendung der erfindungsgemäßen pharmazeutischen Zusammensetzung zur Prophylaxe und/oder Behandlung von Erkrankungen, insbesondere von thromboembolischen Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefen venösen Thrombosen.

Die Erfindung wird nachstehend durch bevorzugte Ausführungsbeispiele näher erläutert. Soweit nicht anders angegeben, beziehen sich nachstehend alle Mengenangaben auf Gewichtsprozente.

### Experimenteller Teil

### 1. Tablettenherstellung mit Granulaten enthaltend den Wirkstoff (I) in hydrophilisierter Form / Wirbelschlcht-granulationseverfahren

### 1.1 Tablettenzusammensetzung (in mg/Tablette)

| | |
|---|---|
| Wirkstoff (I), mikronisiert | 20.0 mg |
| Mikrokristalline Cellulose | 35.0 mg |
| Laktose Monohydrat | 22.9 mg |
| Croscarmellose (Ac-Di-Sol^{®}, FMC) | 3.0 mg |
| Hydroxypropylmethylcellulose, 5 mPa·s | 3.0 mg |
| Natriumlaurylsulfat | 0.5 mg |
| Magnesiumstearat | 0.6 mg |
| Hydroxypropylmethylcellulose, 15 mPa·s | 1.5 mg |
| Polyethylenglykol 3.350 | 0.5 mg |
| Titandioxid | 0.5 mg |
| | 87.5 mg |

### 1.2 Herstellung

Hydroxypropylmethylcellulose (5 mPa·s) und Natriumlaurylsulfat werden in Wasser gelöst In diese Lösung wird der mikronisierte Wirkstoff (I) suspendiert. Die so hergestellte Suspension wird als Granulierflüssiakeit im Rahmen einer Wirbelschichtgranulation auf die Vorlage aus mikrokristalliner Cellulose, Laktose Monohydrat und Croscarmellose aufgesprüht. Nach Trocknung und Siebung (0.8 mm Maschenweite) des entstandenen Granulates wird Magnesiumstearat zugegeben und gemischt. Die so erhaltene pressfertige Mischung wird zu Tabletten mit 6 mm Durchmesser und einer Bruchfestigkeit von 50 - 100 N verpresst. Die anschließende Lackierung der Tabletten erfolgt mit Titandioxid, das in einer wässrigen Lösung aus Hydroxypropylmethylcellulose (15 mPa·s) und Polyethylenglykol suspendiert ist.

### 2. Herstellung von Granulaten enthaltend den Wirkstoff (I) in hydrophilisterter Form und Abfüllung als Sachets

### 2.1 Granulatzusammensetzung (in mg/Sachet)

| | |
|---|---|
| Wirkstoff (I), mikronisiert | 50.0 mg |
| Mannitol | 662.0 mg |
| Croscarmellose (Ac-Di-Sol^{®}, FMC) | 15.0 mg |
| Hydroxypropylmethylcellulose, 5 mPas | 15.0 mg |
| Natriumlaurylsulfat | 1.0 mg |
| Hochdisperses Siliciumdioxid (Aerosil^{®}200, Degussa) | 2.0 mg |
| Erdbeeraaroma, sprühgetrocknet | 5,0 mg |
| | 750.0 mg |

### 2.2 Herstellung

Hydroxypropylmethylcellulose (5 mPa·s) und Natriumlaurylsulfat worden in Wasser gelöst. In diese Lösung wird der mikronisierte Wirkstoff (I) suspendiert. Die so hergestellte Suspension wird als Granulierflüssigkeit im Rahmen einer Wirbelschichtgranulation auf die Vorlage aus Mannitol und Croscarmellose aufgesprüht Nach Trocknung und Siebung (0.8 mm Maschenweite) des entstandenen Granulates werden hochdisperses Siliciumdioxid (Aerosil^{®}) und Erdbeeraroma zugegeben und gemischt. Die so erhaltene Mischung wird zu jeweils 750 mg mit Hilfe einer Sachetabfüllmaschine in Sachetbeutel abgefüllt.

### 3. Herstellung von Granulaten enthaltend den Wirkstoff (I) in hydrophilisierter Form und Abfüllung in Hartgelatinekapseln

### 3.1 Granulatzusammensetzung (in mg/Kapsel)

| | |
|---|---|
| Wirkstoff (I), mikronisiert | 20.0 mg |
| Mikrokristalline Cellulose | 30.0 mg |
| Laktose Monohydrat | 79.5 mg |
| Maisstärke | 25.0 mg |
| Hydroxypropylmethylcellulose, 5 mPas | 4.5 mg |
| Natriumlaurylsulfat | 0.5 mg |
| Hochdisperses Siliciumdioxid (Aerosil^{®} 200, Degussa) | 0.5 mg |
| | 160.0 mg |

### 3.2 Herstellung

Hydroxypropylmethylcellulose (5 mPas) und Natriumlaurylsulfat werden in Wasser gelöst In diese Lösung wird der mikronisierte Wirkstoff (I) suspendiert. Die so hergestellte Suspension wird als Granulierflüssigkeit im Rahmen einer Wirbelschichtgranulation auf die Vorlage aus mikrokristalliner Cellulose, Laktose Monohydrat und Maisstärke aufgesprüht. Nach Trocknung und Siebung (0.8 mm Maschenweite) des entstandenen Granulates wird hochdisperses Siliciumdioxid (Aerosil^{®}) zugegeben und gemischt. Die erhaltene Mischung wird zu jeweils 160 mg in Hartgelatinekapseln der Kapselgröße 2 abgefüllt

### 4. Vergleich von Tabletten mit / ohne hydrophilisiertem Wirkstoff (I)

### 4.1 Tablettenzusammensetzung, -herstellung

Um die Tabletteneigenschaften und die verbesserte Bioverfügbarkeit von Formulierungen mit hydrophilisiertem Wirkstoff (I) zu untersuchen, werden unlackierte Tabletten mit 10 mg Wirkstoffgehalt (I) folgender Zusammensetzung hergestellt (in mg/Tablette):

| | |
|---|---|
| Wirkstoff (I), mikronisiert | 10.0 mg |
| Mikrokristalline Cellulose | 40.0 mg |
| Laktose Monohydrat | 27.9 mg |
| Croscarmellose (Ac-Di-Sol^{®}, FMC) | 3.0 mg |
| Hydroxypropylmethylcellulose, 5 mPas | 3.0 mg |
| Natriumlaurylsulfat | 0.5 mg |
| Magnesiumstearat | 0.6 mg |
| | 85.0 mg |

Tablette A: hergestellt durch Direkttablettierung ohne Granulation
Tablette B: hergestellt durch das unter 1.2 beschriebene Wirbelschicht-granulations-/Suspensionsverfahren

Die Mischung für Tablette A und das Granulat für Tablette B werden jeweils zu Tabletten mit einem Durchmesser von 6 mm und einer Bruchfestigkeit von ca. 70 - 80 N gepresst.

### 4.2 Tabletteneigenschaften

### 4.2.1 Zerfallszeit in Wasser (USP-Zerfallstester, Erweka):

- Tablette A:: ca. 1.5 Minuten
- Tablette B:: ca. 6.5 Minuten

### 4.2.2 in-vitro Freisetzung

In der folgenden Tabelle 1 sind die freigesetzten Wirkstoffmengen bezogen auf den deklarierten Gesamtgehalt der Tabletten wiedergegeben:

**Tabelle 1: in-vitro Freisetzung**

| | 15 min | 30 min | 45 min | 60 min |
|---|---|---|---|---|
| Tablette A | 87 % | 92 % | 93 % | 94 % |
| Tablette B | 94 % | 95 % | 96 % | 96 % |

### (USP-Paddle, 900 ml Acetat-Puffer pH 4.5 + 0.5 % Natriumlaurylsulfat, 75 UpM)

### 4.2.3 Bioverfügbarkeit

Zur Untersuchung der Bioverfügbarkeit wurden drei Hunden jeweils drei Tabletten A bzw. drei Tabletten B cross-over appliziert. In der folgenden Tabelle 2 sind die entsprechenden pharmakokinetischen Parameter nach oraler Gabe von 3 mg Wirkstoff (I) / kg aufgelistet:

**Tabelle 2: Pharmakoklnetische Parameter von Wirkstoff (I)**

| | | Tier | | | Mean geom. | S.D. geom. | Mean arithm. | S.D. arithm. |
|---|---|---|---|---|---|---|---|---|
| | | 1 | 2 | 3 | | | | |
| **Tablette A** | | | | | | | | |
| AUC(0-24) | [mg·h/L] | 1,39 | 2,31 | 3,34 | 2,21 | 1,55 | 2,35 | 0,974 |
| **AUC (0-24)ₙₒᵣₘ** | **[kg·h/L]** | **0,464** | **0,770** | **1,11** | **0,735** | **1,55** | **0,782** | **0,325** |
| Cₘₐₓ | [mg/L] | 0,299 | 0,398 | 0,430 | 0,371 | 1,21 | 0,376 | 0,0684 |
| C_{max,norm} | [kg/L] | 0,0997 | 0,133 | 0,143 | 0,124 | 1,21 | 0,125 | 0,0228 |
| C(24)/Cₘₐₓ | [%] | 12,2 | 2,99 | 55,1 | 12,6 | 4,29 | 23,4 | 27,8 |
| tₘₐₓ | [h] | 1,00 | 1,50 | 0,750 | 1,04 | 1,42 | 1,08 | 0,382 |

| **Tablette B** | | | | | | | | |
|---|---|---|---|---|---|---|---|---|
| AUC(0-24) | [mg-h/L] | 2,82 | 3,03 | 3,73 | 3,17 | 1,16 | 3,19 | 0,476 |
| **AUC (0-24)ₙₒᵣₘ** | **[kg·h/L]** | **0,938** | **1,01** | **1,24** | **1,06** | **1,16** | **1,06** | **0,159** |
| Cₘₐₓ | [mg/L] | 0,478 | 0,513 | 0,321 | 0,428 | 1,29 | 0,437 | 0,102 |
| C_{max,norm} | [kg/L] | 0,159 | 0,171 | 0,107 | 0,143 | 1,29 | 0,146 | 0,0341 |
| C(24)/Cₘₐₓ | [%] | 26,4 | 1,17 | 93,4 | 14,2 | 9,53 | 40,3 | 47,7 |
| tₘₐₓ | [h] | 1,00 | 1,50 | 0,750 | 1,04 | 1,42 | 1,08 | 0,382 |

Ergebnis: Trotz langsameren Zerfalls (siehe 4.2.1) und sehr ähnlicher in-vitro Freisetzung (siehe 4.2.2) von Tablette B im Vergleich zu Tablette A besitzt Tablette B deutliche Vorteile bei der Absorption und damit eine um ca. 35 % gesteigerte Bioverfügbarkeit. Gleichzeitig ist eine deutliche Abnahme der Variabilität festzustellen. Der einzige Unterschied zwischen Tablette A und Tablette B ist die Hydrophilisierung des Wirkstoffes (I) bei Tablette B mit Hilfe des Suspensionsverfahrens im Rahmen der Feuchtgranulierung.

## Patentansprüche

1. Verfahren zur Herstellung einer festen, oral applizierbaren pharmazeutischen Zusammensetzung enthaltend 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) in hydrophilisierter Form, **dadurch gekennzeichnet, dass**
(a) zunächst ein den Wirkstoff (I) in hydrophilisierter Form enthaltendes Granulat durch Feuchtgranulation hergestellt wird
(b) und das Granulat dann, gegebenenfalls unter Zusatz pharmazeutisch geeigneter Zusatzstoffe, in die pharmazeutische Zusammensetzung überführt wird,
wobei als Feuchtgranulationsmethode die Wirbelschichtgranulation verwendet wird.

2. Verfahren gemäß Anspruch 1, **dadurch gekennzeichnet, dass** der Wirkstoff (I) in kristalliner Form eingesetzt wird.

3. Verfahren gemäß Anspruch 2, **dadurch gekennzeichnet, dass** der Wirkstoff (I) in mikronisierter Form eingesetzt wird.

4. Verfahren gemäß einem der Ansprüche 1 bis 3, **dadurch gekennzeichnet, dass** der Wirkstoff (I) in der Granulierflüssigkeit suspendiert in die Feuchtgranulation eingetragen wird.

5. Verfahren gemäß einem der Ansprüche 1 bis 4, **dadurch gekennzeichnet, dass** die pharmazeutische Zusammensetzung eine den Wirkstoff (I) schnell freisetzende Tablette ist.

6. Feste, oral applizierbare pharmazeutische Zusammensetzung hergestellt durch das Verfahren gemäß Anspruch 1.

7. Feste, oral applizierbare pharmazeutische Zusammensetzung, enthaltend 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) in durch ein Verfahren nach Anspruch 1 herstellbarer hydrophilisierter Form.

8. Pharmazeutische Zusammensetzung gemäß Anspruch 7, enthaltend den Wirkstoff (I) in kristalliner Form.

9. Pharmazeutische Zusammensetzung gemäß Anspruch 8, enthaltend den Wirkstoff (I) in mikronisierter Form.

10. Pharmazeutische Zusammensetzung gemäß einem der Ansprüche 6 bis 9, **dadurch gekennzeichnet, dass** der Wirkstoff (I) in einer Konzentration von 1 bis 60 % bezogen auf die Gesamtmasse der Formulierung vorliegt.

11. Pharmazeutische Zusammensetzung gemäß einem der Ansprüche 6 bis 10, enthaltend Natriumlaurylsulfat als Netzmittel.

12. Pharmazeutische Zusammensetzung gemäß Anspruch 11, enthaltend Natriumlaurylsulfat in einer Konzentration von 0.1 bis 5 %, bezogen auf die Gesamtmasse.

13. Pharmazeutische Zusammensetzung gemäß einem der Ansprüche 6 bis 12, enthaltend Hydroxypropylmethylcellulose als hydrophiles Bindemittel.

14. Pharmazeutische Zusammensetzung gemäß Anspruch 13, enthaltend 14. Hydroxypropylmethylcellulose in einer Konzentration von 1 bis 15 %, bezogen auf die Gesamtmasse.

15. Pharmazeutische Zusammensetzung gemäß einem der Ansprüche 6 bis 14 in Form einer Tablette.

16. Pharmazeutische Zusammensetzung gemäß Anspruch 15 in Form einer schnell freisetzenden Tablette.

17. Pharmazeutische Zusammensetzung gemäß Anspruch 15 oder 16, **dadurch gekennzeichnet, dass** die Tablette mit einem Lack überzogen ist.

18. Verwerdung der pharmazeutischen Zusammensetzung gemäß einem der Ansprüche 6 bis 17 zur Herstellung eines Arzneimittels zur Prophylaxe und/oder Behandlung von thromboembolischen Erkrankungen.

19. Verwendung von 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) in hydrophilisierter Form zur Herstellung einer festen oral applizierbaren pharmazeutischen Zusammensetzung nach Anspruch 6 oder 7 zur Prophylaxe und/oder Behandlung von thromboembolischen Erkrankungen.

## Claims

1. Method for the production of a solid, orally administrable pharmaceutical composition comprising 5-chloro-*N*-({(5S)-2-oxo-3-[4-(3-oxo-4-morpholinyl)phenyl]-1,3-oxazolidin-5-yl}-methyl)-2-thiophenecarboxamide (I) in hydrophilized form, **characterized in that**
(a) firstly granules comprising the active ingredient (I) in hydrophilized form are produced by wet granulation
(b) and the granules are then converted to the pharmaceutical composition, optionally with the addition of pharmaceutically suitable additives,
where the wet granulation method used is fluidized-bed granulation.

2. Method according to Claim 1, **characterized in that** the active ingredient (I) is used in crystalline form.

3. Method according to Claim 2, **characterized in that** the active ingredient (I) is used in micronized form.

4. Method according to one of Claims 1 to 3, **characterized in that** the active ingredient (I) is introduced into the wet granulation suspended in the granulation liquid.

5. Method according to one of Claims 1 to 4, **characterized in that** the pharmaceutical composition is a tablet that rapidly releases the active ingredient (I).

6. Solid, orally administrable pharmaceutical composition produced by the method according to Claim 1.

7. Solid, orally administrable pharmaceutical composition comprising 5-chloro-N-({(5S)-2-oxo-3-[4-(3-oxo-4-morpholinyl)phenyl]-1,3-oxazolidin-5-yl}-methyl)-2-thiophene-carboxamide (I) in hydrophilized form producible by a method according to Claim 1.

8. Pharmaceutical composition according to Claim 7, comprising the active ingredient (I) in crystalline form.

9. Pharmaceutical composition according to Claim 8, comprising the active ingredient (I) in micronized form.

10. Pharmaceutical composition according to one of Claims 6 to 9, **characterized in that** the active ingredient (I) is present in a concentration of from 1 to 60%, based on the total mass of the formulation.

11. Pharmaceutical composition according to one of Claims 6 to 10, comprising sodium lauryl sulphate as wetting agent.

12. Pharmaceutical composition according to Claim 11, comprising sodium lauryl sulphate in a concentration of from 0.1 to 5%, based on the total mass.

13. Pharmaceutical composition according to one of Claims 6 to 12, comprising hydroxypropylmethylcellulose as hydrophilic binder.

14. Pharmaceutical composition according to Claim 13, comprising hydroxypropylmethylcellulose in a concentration of from 1 to 15%, based on the total mass.

15. Pharmaceutical composition according to one of Claims 6 to 14 in the form of a tablet.

16. Pharmaceutical composition according to Claim 15 in the form of a rapidly releasing tablet.

17. Pharmaceutical composition according to Claim 15 or 16, **characterized in that** the tablet is covered with a coating.

18. Use of the pharmaceutical composition according to one of Claims 6 to 17 for producing a medicament for the prophylaxis and/or treatment of thromboembolic disorders.

19. Use of 5-chloro-N-({(5S)-2-oxo-3-[4-(3-oxo-4-morpholinyl)phenyl]-1,3-oxazolidin-5-yl}-methyl)-2-thiophenecarboxamide (I) in hydrophilized form for producing a solid, orally administrable pharmaceutical composition according to Claim 6 or 7 for the prophylaxis and/or treatment of thromboembolic disorders.

## Revendications

1. Procédé pour la préparation d'une composition pharmaceutique solide, administrable par voie orale, contenant du 5-chloro-N-({(5S)-2-oxo-3-[4-(3-oxo-4-morpholinyl)-phényl]-1,3-oxazolidin-5-yl}-méthyl)-2-thiophènecarboxamide (I) sous forme hydrophilisée, **caractérisé en ce que**
(a) on prépare d'abord un granulat contenant la substance active (I) sous forme hydrophilisée par granulation humide,
(b) et on transforme ensuite le granulat, le cas échéant avec addition d'additifs pharmaceutiquement appropriés, en composition pharmaceutique,
la granulation en couche tourbillonnante étant utilisée comme procédé de granulation humide.

2. Procédé selon la revendication 1, **caractérisé en ce que** la substance active (I) est utilisée sous forme cristalline.

3. Procédé selon la revendication 2, **caractérisé en ce que** la substance active (I) est utilisée sous forme micronisée.

4. Procédé selon l'une quelconque des revendications 1 à 3, **caractérisé en ce que** la substance active (I) est introduite dans la granulation humide sous forme mise en suspension dans le liquide de granulation.

5. Procédé selon l'une quelconque des revendications 1 à 4, **caractérisé en ce que** la composition pharmaceutique est un comprimé libérant rapidement la substance active (I).

6. Composition solide, administrable par voie orale, préparée par le procédé selon la revendication 1.

7. Composition pharmaceutique solide, administrable par voie orale, contenant du 5-chloro-N-({(5S)-2-oxo-3-[4-(3-oxo-4-morpholinyl)-phényl]-1,3-oxazolidin-5-yl}-méthyl)-2-thiophènecarboxamide (I) sous une forme hydrophilisée pouvant être préparée par le procédé selon la revendication 1.

8. Composition pharmaceutique selon la revendication 7, contenant la substance active (I) sous forme cristalline.

9. Composition pharmaceutique selon la revendication 8, contenant la substance active (I) sous forme micronisée.

10. Composition pharmaceutique selon l'une quelconque des revendications 6 à 9, **caractérisée en ce que** la substance active (I) se trouve en une concentration de 1 à 60%, par rapport à la masse totale de la formulation.

11. Composition pharmaceutique selon l'une quelconque des revendications 6 à 10, contenant du laurylsulfate de sodium comme agent mouillant.

12. Composition pharmaceutique selon la revendication 11, contenant du laurylsulfate de sodium en une concentration de 0,1 à 5%, par rapport à la masse totale.

13. Composition pharmaceutique selon l'une quelconque des revendications 6 à 12, contenant de l'hydroxypropylméthylcellulose comme liant hydrophile.

14. Composition pharmaceutique selon la revendication 13, contenant de l'hydroxypropylméthylcellulose en une concentration de 1 à 15%, par rapport à la masse totale.

15. Composition pharmaceutique selon l'une quelconque des revendications 6 à 14 sous forme d'un comprimé.

16. Composition pharmaceutique selon la revendication 15 sous forme d'un comprimé à libération rapide.

17. Composition pharmaceutique selon la revendication 15 ou 16, **caractérisée en ce que** le comprimé est revêtu par une laque.

18. Utilisation de la composition pharmaceutique selon l'une quelconque des revendications 6 à 17 pour la préparation d'un médicament pour la prophylaxie et/ou le traitement de maladies thromboemboliques.

19. Utilisation de 5-chloro-N-({(5S)-2-oxo-3-[4-(3-oxo-4-morpholinyl)-phényl]-1,3-oxazolidin-5-yl}-méthyl)-2-thiophènecarboxamide (I) sous forme hydrophilisée pour la préparation d'une composition pharmaceutique solide, administrable par voie orale selon la revendication 6 ou 7 pour la prophylaxie et/ou le traitement de maladies thromboemboliques.
